# EUROPÄISCHE PATENTANMELDUNG

(11) **EP 1 074 298 A2**
(43) Veröffentlichungstag der Anmeldung: **07.02.2001**
(21) Anmeldenummer: 00115124.0
(22) Anmeldetag: 12.07.2000
(51) Int. Cl.: B01J 21/06, B01J 23/62, C07C 5/333

(54) **Oxidkatalysatoren enthaltend zumindest Silika und Gruppe IVB Oxid**

(30) Priorität: 06.08.1999 DE 19937105
(71) Anmelder: BASF AKTIENGESELLSCHAFT, 67056 Ludwigshafen (DE)
(72) Erfinder: Heineke, Daniel, Dr., 67487 Maikammer (DE); Harth, Klaus, Dr., 67317 Altleiningen (DE); Stabel, Uwe, Dr., 67166 Otterstadt (DE); Hofstadt, Otto, 67122 Altrip (DE)

(57) **Zusammenfassung**

Katalysatoren, die
a) 10 bis 99,9 Gew.-% Zirkondioxid und/oder Titandioxid und
b) 0,1 bis 30 Gew.-% Siliciumdioxid und
c) 0 bis 60 Gew.-% Aluminiumoxid und
d) 0,1 bis 10 Gew.-% mindestens eines Elementes der ersten oder zweiten Hauptgruppe, eines Elementes der dritten Nebengruppe, eines Elementes der achten Nebengruppe des Periodensystems der Elemente und/oder Zinn enthalten,
mit der Maßgabe, daß die Summe der Gewichtsprozente 100 ergibt, ein Verfahren zur Dehydrierung von C₂- bis C₁₆-Kohlenwasserstoffen und die Verwendung dieser Katalysatoren dazu sowie ein Verfahren zur Herstellung dieser Katalysatoren.

## Beschreibung

Die vorliegende Erfindung betrifft Katalysatoren, die a) Zirkondioxid und/oder Titandioxid und b) Siliciumdioxid und c) gegebenenfalls Aluminiumoxid und d) mindestens ein Element der ersten oder zweiten Hauptgruppe, ein Element der dritten Nebengruppe, ein Element der achten Nebengruppe des Periodensystems der Elemente und/oder Zinn enthalten.

Aus der US-A-5,220,091 sind Katalysatoren bestehend aus Pt/Sn als Aktivkomponente auf einem Zn-Spinell-Träger zur Dehydrierung von kleinen Kohlenwasserstoffmolekülen wie Isobutan mit Wasserdampf als Verdünnungsmittel bekannt. Hinsichtlich ihrer Performance sind diese Katalysatoren verbesserungsbedürftig, denn es werden trotz hoher Verdünnung des Feed mit Wasserdampf (Verhältnis 4:1) bei hohen Reaktionstemperaturen von 600°C nur relativ geringe Umsätze und Selektivitäten erzielt. Ebenfalls verbesserungswürdig ist die Standzeit der Katalysatoren, denn es muß nach einer Betriebszeit von nur 7 h regeneriert werden.

Aus der US-A-4,788,371 sind Pt/Sn/Cs/Al₂O₃-Katalysatoren zur Dehydrierung von Kohlenwasserstoffen in Gegenwart von Wasserdampf (z.B. Wasserdampf/Propan 10:1) bekannt. Trotz des hohen Verdünnungsgrades werden nur geringe Umsätze von 21% erreicht.

Aus der WO-A-94/29021 sind Katalysatoren auf der Basis von Mischoxiden von Magnesium und Aluminium mit einem Edelmetall der Gruppe VIII, einem Metall der Gruppe IVa und gegebenenfalls einem Alkalimetall der Gruppe Ia des Periodensystems der Elemente zur Dehydrierung z.B. eines Gasgemisches aus H₂O/Propan/H₂/N₂ im Verhältnis 8:7:1:5 bekannt. Nachteilig für eine technische Anwendung dieser Katalysatoren ist ihre geringe Härte, die einen technischen Einsatz schwierig macht. Weiterhin sind diese Katalysatoren in ihrer Performance, insbesondere bei niedrigen Reaktionstemperaturen verbesserungsbedürftig. Ein weiterer Nachteil ist die aufwendige Fahrweise, die zur Erhaltung der Performance den Zusatz von Wasserstoff zum Feed und die Zumischung von Stickstoff zur weiteren Verdünnung erfordert.

Der vorliegenden Erfindung lag deshalb die Aufgabe zugrunde, den zuvor genannten Nachteilen abzuhelfen.

Demgemäß wurden neue und verbesserte Katalysatoren gefunden, die
a) 10 bis 99,9 Gew.-% Zirkondioxid und/oder Titandioxid und
b) 0,1 bis 30 Gew.-% Siliciumdioxid und
c) 0 bis 60 Gew.-% Aluminiumoxid und
d) 0,1 bis 10 Gew.-% mindestens eines Elementes der ersten oder zweiten Hauptgruppe, eines Elementes der dritten Nebengruppe, eines Elementes der achten Nebengruppe des Periodensystems der Elemente und/oder Zinn enthalten,
mit der Maßgabe, daß die Summe der Gewichtsprozente 100 ergibt, ein Verfahren zur Dehydrierung von C₂- bis C₁₆-Kohlenwasserstoffen und die Verwendung dieser Katalysatoren dazu sowie ein Verfahren zur Herstellung dieser Katalysatoren.

Die erfindungsgemäßen Katalysatoren enthalten, bevorzugt bestehen aus
a) 10 bis 99,9 Gew.-%, bevorzugt 20 bis 98 Gew.-%, besonders bevorzugt 30 bis 95 Gew.-% Zirkondioxid und/oder Titandioxid, bevorzugt in Form von Rutil oder Anatas oder deren Gemische, besonders bevorzugt Zirkondioxid und/oder Titandioxid, insbesondere Zirkondioxid und
b) 0,1 bis 30 Gew.-%, bevorzugt 0,5 bis 25 Gew.-%, besonders bevorzugt 2 bis 10 Gew.-% Siliciumdioxid und
c) 0 bis 60 Gew.-%, bevorzugt 0,1 bis 50 Gew.-%, besonders bevorzugt 1 bis 40 Gew.-%, insbesondere 5 bis 30 Gew.-% Aluminiumoxid und
d) 0,1 bis 10 Gew.-%, bevorzugt 0,15 bis 8 Gew.-%, besonders bevorzugt 0,2 bis 5 Gew.-%, mindestens eines Elementes der ersten oder zweiten Hauptgruppe, eines Elementes der dritten Nebengruppe, eines Element der achten Nebengruppe des Periodensystems der Elemente und/oder Zinn enthalten,
wobei sich die Summe der Gewichtsprozente zu 100 ergibt.

Der Gehalt der erfindungsgemäßen Katalysatoren an einem Edelmetall als dehydrieraktive Komponente beträgt in der Regel 0 bis 5 Gew.-%, bevorzugt 0,1 bis 1 Gew.-%, besonders bevorzugt 0,2 bis 0,5 Gew.-%.

Zur Herstellung der erfindungsgemäßen Katalysatoren können Precursoren der Oxide des Zirkons, Titans, Siliciums und Aluminiums, die sich durch Caicinieren in die Oxide umwandeln lassen, eingesetzt werden. Diese können nach bekannten Verfahren, zum Beispiel nach dem Sol-Gel-Verfahren, Fällung der Salze, Entwässern der entsprechenden Säuren, Trockenmischen, Aufschlämmen oder Sprühtrocknen hergestellt werden. Zum Beispiel kann zur Herstellung eines ZrO₂ · xAl₂O₃ · xSiO₂-Mischoxides zunächst ein wasserreiches Zirkonoxid der allgemeinen Formel ZrO₂ · H₂O durch Fällung eines geeigneten Zirkon-haltigen Precursors hergestellt werden. Geeignete Precursoren des Zirkons sind zum Beispiel Zr(NO₃)₄, ZrOCl₂, oder ZrCl₄. Die Fällung selbst erfolgt durch Zugabe einer Base wie zum Beispiel NaOH, KOH, Na₂CO₃ und NH₃ und ist beispielsweise in der EP-A-849 224 beschrieben.

Zur Herstellung eines ZrO₂ · xSiO₂-Mischoxides kann der zuvor erhaltene Zr-Precursor mit einem Si-haltigen Precursor gemischt werden. Gut geeignete Precursoren des SiO₂ sind zum Beispiel wasserhaltige Sole des SiO₂ wie Ludox™. Die Mischung der beiden Komponenten kann beispielsweise durch einfaches mechanisches Vermischen oder durch Sprühtrocknen in einem Sprühturm erfolgen.

Zur Herstellung eines ZrO₂ · xSiO₂ · xAl₂O₃-Mischoxides kann die wie oben beschrieben erhaltene SiO₂ · ZrO₂-Pulvermischung mit einem Al-haltigen Precursor versetzt werden. Dies kann zum Beispiel durch einfaches mechanisches Mischen in einem Kneter erfolgen. Die Herstellung eines ZrO₂ · xSiO₂ · xAl₂O₃-Mischoxides kann aber auch in einem einzigen Schritt durch Trockenmischung der einzelnen Precursoren erfolgen.

Die Mischoxide haben gegenüber reinem ZrO₂ unter anderem den Vorteil, daß sie sich leicht verformen lassen. Dazu wird die erhaltene Pulvermischung im Kneter gegebenenfalls mit einer konzentrierten Säure versetzt und kann dann in einen Formkörper, z.B. mittels einer Strangpresse oder eines Extruders überführt werden.

Ein erfindungsgemäßer Vorteil ist die gezielte Beeinflussung der Porenstruktur durch die Verwendung von Mischoxiden. Die Korngröße der verschiedenen Precursoren beeinflussen das Porengefüge. So lassen sich beispielsweise über die Verwendung von Al₂O₃ mit einem geringen Glühverlust und einer definierten Korngrößenzusammensetzung Makroporen im Gefüge erzeugen. Bewährt hat sich in diesem Zusammenhang die Verwendung von Puralox (Al₂O₃ mit einem Glühverlust von etwa 3 %).

Die Mischoxid-Träger der erfindungsgemäßen Katalysatoren weisen nach der Calcinierung im allgemeinen höhere BET-Oberflächen als reine ZrO₂-Träger auf. Die BET-Oberflächen der Mischoxid-Träger liegen im allgemeinen zwischen 40 und 300 m²/g, bevorzugt zwischen 50 und 200 m²/g, besonders bevorzugt zwischen 80 und 150 m²/g. Das Porenvolumen der erfindungsgemäßen Katalysatoren beträgt üblicherweise 0,1 bis 0,8 ml/g, bevorzugt 0,2 bis 0,6 ml/g. Der durch Hg-Porosimetrie bestimmbare mittlere Porendurchmesser der erfindungsgemäßen Katalysatoren liegt zwischen 5 und 20 nm, bevorzugt zwischen 8 und 18 nm.

Die Calcinierung der Mischoxid-Träger erfolgt zweckmäßigerweise nach dem Aufbringen der Aktivkomponenten und wird bei Temperaturen von 400 bis 700°C, bevorzugt von 500 bis 650°C, besonders bevorzugt bei 560 bis 620°C durchgeführt. Die Calciniertemperatur sollte dabei üblicherweise mindestens so hoch sein wie die Reaktionstemperatur der Dehydrierung für die die erfindungsgemäßen Katalysatoren eingesetzt werden.

Die Dotierung der Mischoxid-Träger mit einer basischen Verbindung kann entweder während der Träger-Herstellung, zum Beispiel durch gemeinsame Fällung oder nachträglich zum Beispiel durch Tränken des Mischoxides mit einer Alkali- oder Erdalkalimetallverbindung oder einer Verbindung der 3. Nebengruppe oder einer Seltenerdmetall-Verbindung erfolgen. Besonders geeignet zur Dotierung sind K, Cs und Lanthan.

Die Aufbringung der dehydrieraktiven Komponente, das üblicherweise ein Metall der VIII. ist, erfolgt in der Regel durch Tränkung mit einem geeignetem Metallsalzprecursor, der sich durch Calcinieren in das entsprechende Metalloxid umwandeln läßt. Statt durch Tränkung kann die dehydrieraktive Komponente aber auch durch andere Verfahren wie beispielsweise Aufsprühen des Metallsalzprecursors erfolgen. Geeignete Metallsalzprecursoren sind z.B. die Nitrate, Acetate und Chloride der entsprechenden Metalle, möglich sind auch komplexe Anionen der verwendeten Metalle. Bevorzugt werden Platin als H₂PtCl₆ oder Pt(NO₃)₂ eingesetzt. Als Lösungsmittel für die Metallsalzprecursoren eignen sich Wasser genauso wie organische Lösungsmittel. Besonders geeignet sind niedere Alkohole wie Methanol und Ethanol.

In einer bevorzugten Ausführungsform wird der Katalysator nach folgenden Schritten hergestellt:
a) Versprühen oder Trockenmischen geeigneter Verbindungen der Elemente des Zirkons und/oder Titans und Siliciums. Geeignete Verbindungen sind zum Beispiel wasserhaltiges ZrO₂ · xH₂O, welches durch Fällung aus Precursoren wie Zr(NO₃)₄, ZrOCl₂ oder Zr(OR)₄ erhalten wurde und zum Beispiel SiO₂-Sol (Ludox™). Anschließendes Calcinieren bei Temperaturen im Bereich von 400 bis 700°C, bevorzugt 550 bis 650°C.
b) Vermischen der aus Schritt a) erhaltenen Zusammensetzung mit einer Verbindung des Aluminiums, z.B. Al₂O₃ oder AlO(OH) (Böhmit) unter Zusatz einer Säure wie konz. HNO₃ oder konz. HCOOH.
c) Überführung der aus b erhaltenen Masse in einen Formkörper, z.B. durch Verstrangen, Tablettieren oder Extrudieren.
d) Tränkung des erhaltenen Formkörpers mit einer Platin- und Zinn-haltigen Lösung, Trocknung und Calcinierung bei Temperaturen von 400 bis 650°C. Bevorzugt eignet sich eine gemeinsame Lösung von H₂PtCl₆ und SnCl₂ in Ethanol.
e) Tränkung mit einer Kalium- und Cäsium-haltigen wäßrigen Lösung, die weitere basische Elemente wie Lanthan enthalten kann. Trocknung und Calcinierung bei Temperaturen von 400 bis 650°C.

Geeignete Precursoren bei der Verwendung von Edelmetallen als dehydrieraktive Komponente sind auch die entsprechenden Edelmetallsole, die nach einem der bekannten Verfahren, zum Beispiel durch Reduktion eines Metallsalzes in Gegenwart eines Stabilisators wie PVP mit einem Reduktionsmittel hergestellt werden können. Die Herstelltechnik wird in der deutschen Patentanmeldung DE-A-195 00 366 ausführlich behandelt.

Der Katalysator kann im Reaktor fest angeordnet oder z.B. in Form eines Wirbelbettes verwendet werden und eine entsprechende Gestalt haben. Geeignet sind z.B. Formen wie Splitt, Tabletten, Monolithen, Kugeln, oder Extrudate (Stränge, Wagenräder, Sterne, Ringe).

Als Alkali- und Erdalkalimetallprecursor verwendet man in der Regel Verbindungen, die sich durch Calcinieren in die entsprechenden Oxide umwandeln lassen. Geeignet sind zum Beispiel Hydroxide, Carbonate, Oxalate, Acetate oder gemischte Hydroxycarbonate der Alkali- und Erdalkalimetalle.

Wird der Mischoxid-Träger zusätzlich oder ausschließlich mit einem Metall der dritten Nebengruppe dotiert, so sollte man auch in diesem Fall von Verbindungen ausgehen, die sich durch Calcinieren in die entsprechenden Oxide umwandeln lassen. Wird Lanthan verwendet, so sind beispielsweise Lanthan-Oxid-Carbonat, La(OH)₃, La₃(CO₃)₂, La(NO₃)₃ oder Lanthanverbindungen die organische Anionen enthalten, wie La-Acetat, La-Formiat, oder La-Oxalat geeignet.

Die Propan-Dehydrierung wird in der Regel bei Reaktionstemperaturen von 300 bis 800°C, bevorzugt 450 bis 700°C, und einem Druck von 0,1 bis 100 bar, bevorzugt 0,1 bis 40 bar mit einer GHSV (Gas Hourly Space Velocity) von 100 bis 10.000 h⁻¹, bevorzugt 500 bis 2.000 h⁻¹ durchgeführt. Neben dem zu dehydrierenden Kohlenwasserstoff können im Feed Verdünnungsmittel wie beispielsweise CO₂, N₂, Edelgase und/oder Dampf (Wasserdampf), bevorzugt N₂ und/oder Dampf, besonders bevorzugt Dampf zugegen sein.

Ein spezielles Merkmal der erfindungsgemäßen Katalysatoren ist, daß die Katalysatoren bei der Dehydrierung von Kohlenwasserstoffen in Gegenwart von Wasserdampf aktiv sind und die damit verbundenen Vorteile wie Aufhebung der Gleichgewichtslimitierung, Verringerung der Verkokung und Verlängerung der Standzeiten genutzt werden können.

Gegebenenfalls kann zum Kohlenwasserstoff-Feed Wasserstoff zugegeben werden, wobei das Verhältnis von Wasserstoff zu Kohlenwasserstoffstrom in der Regel 0,1:1 bis 100:1, bevorzugt 1:1 bis 20:1 beträgt. Bevorzugt kann die Dehydrierung von Kohlenwasserstoffen mit den erfindungsgemäßen Katalysatoren ohne den Zusatz von Wasserstoff betrieben werden.

Neben der kontinuierlichen Zugabe eines Gases, insbesondere von Dampf (Wasserdampf), gibt es die Möglichkeit, den Katalysator durch Überleiten von Wasserstoff oder Luft von Zeit zu Zeit zu regenerieren. Die Regenerierung selbst findet bei Temperaturen im Bereich 300 bis 900°C, bevorzugt 400 bis 800°C mit einem freien Oxidationsmittel, vorzugsweise mit Luft oder in reduktiver Atmosphäre, vorzugsweise mit Wasserstoff statt. Die Regenerierung kann bei Unterdruck, Normaldruck (Atmosphärendruck) oder Überdruck betrieben werden. Bevorzugt sind Drücke im Bereich 0,5 bis 100 bar.

Für die Dehydrierung mit den erfindungsgemäßen Katalysatoren eignen sich Kohlenwasserstoffe beispielsweise C₂- bis C₁₆-Kohlenwasserstoffe wie Ethan, n-Propan, n-Butan, iso-Butan, n-Pentan, iso-Pentan, n-Hexan, n-Heptan, n-Octan, n-Nonan, n-Decan, n-Undecan, n-Dodecan, n-Tridecan, n-Tetradecan, n-Pentadecan, n-Hexadecan, bevorzugt C₂- bis C₈-Kohlenwasserstoffe wie Ethan, n-Propan, n-Butan, iso-Butan, n-Pentan, iso-Pentan, n-Hexan, n-Heptan, n-Octan, besonders bevorzugt C₂- bis C₄-Kohlenwasserstoffe wie Ethan, n-Propan, n-Butan und iso-Butan, insbesondere Propan und iso-Butan.

Propylen ist ein gefragtes Produkt, insbesondere zur Synthese von Polypropylen oder zur Synthese von funktionalisierten Monomeren und deren Polymerisationsprodukten. Eine Alternative zur Herstellung von Propylen durch Steamcracking von leichtem Naphtha ist die Dehydrierung von Propan.

Isobuten ist ein wichtiges Produkt, insbesondere zur Herstellung von MTBE (Methyl-tert.-butyl-ether). Es wird vor allem in den USA als Kraftstoffadditiv zur Erhöhung der Oktanzahl verwendet. Isobuten läßt sich analog zu Propylen durch Dehydrierung von Isobutan herstellen.

### Beispiele

### Katalysatorherstellung

### Beispiel 1

537,31 g Zr(OH)₄ · xH₂O mit einem ZrO₂-Gehalt von 89,8% wurden in 2000 ml Wasser aufgeschlämmt und mit einem ULTRA-TURRAX™ T 50 (Firma Ika) zerkleinert. Zu dieser Suspension wurde ein SiO₂-Sol (Ludox™) mit einem SiO₂-Gehalt von 47,6% gegeben. Die Sprühmaische wurde bei einem Feststoff/Wasser-Verhältnis von 1 : 3,7 in einem NIRO-Atomizer mit einer Kopf-Temperatur von 350°C, einer Ausgangstemperatur von 105 bis 110°C, einem Sprühdruck von 5,2 bar bei 28000 U/min versprüht, so daß ein weißes Pulver entstand. Das Sprühpulver wurde 2 h bei 600°C calciniert. 400 g des Sprühpulvers wurden mit 133,3 g Pural™ SCF (Böhmit mit einem Glühverlust von 25 %) unter Zugabe von 25 g konz. HNO₃ (65 %ig) gemischt und 2,5 h verknetet. Der Knetansatz wurde mittels einer Strangpresse zu 3 mm Vollsträngen bei 70 bar verarbeitet. Die Schnitthärte betrug 37 N/Strang (SA = 11 N).

62 g des zuvor zu 1,6 bis 2 mm gesplitteten Trägers wurden mit einer Lösung, die 0,7208 g SnCl₂ · 2 H₂O und 0,474 g H₂PtCl₆ · 6 H₂O in 369 ml Ethanol enthielt, getränkt. Die überstehende Lösung wurde i. Vak. entfernt und der Rückstand 15 h bei 100°C getrocknet und anschließend 3 h bei 560°C calciniert. Anschließend wurde mit einer Lösung von 0,465 g CsNO₃ und 0,8172 g KNO₃ in 154 ml Wasser getränkt und der Katalysator 15 h bei 100°C getrocknet und anschließend 3 h bei 560°C calciniert.

Der Katalysator wies ein Splitt-Stampfgewicht von 1,18 g/ml auf. Die BET-Oberfläche betrug 84 m²/g. Durch Quecksilber-Porosimetrie-Messungen wurde ein Porenvolumen von 0,26 ml/g ermittelt und eine Porenfläche von 88 m²/g und ein mittlerer Porenradius von 11,0 nm errechnet.

Die Zusammensetzung des Katalysators ist Tabelle 1 zu entnehmen.

### Beispiel 2

Es wurde analog Beispiel 1 verfahren mit den Unterschieden, daß zur Herstellung des Mischoxid Trägers 521,3 g Zr(OH)₄ · xH₂O und 73;53 g SiO₂-Sol (Ludox™) eingesetzt wurden. Das Feststoff/Wasser-Verhältnis betrug beim Versprühen 1 : 4,5 und beim Verkneten wurden 30,22 g konz. HNO₃ (65% ig) eingesetzt. Die Schnitthärte betrug 61 N (SA = 19 N).

Der Katalysator wies ein Splitt-Stampfgewicht von 0,963 g/ml auf. Die BET-Oberfläche betrug 103 m²/g. Durch Quecksilber-Porosimetrie-Messungen wurde ein Porenvolumen von 0,35 ml/g ermittelt und eine Porenfläche von 100 m²/g und ein mittlerer Porenradius von 15,5 nm errechnet.

Die Zusammensetzung des Katalysators ist Tabelle 1 zu entnehmen.

### Beispiel 3

Es wurde analog Beispiel 1 verfahren mit den Unterschieden, daß beim Verkneten 353,36 g des im ersten Schritt erhaltenen Sprühpulvers, 266,66 g Pural™ SCF (Böhmit, Glühverlust 25 %) und 31 g konz. HNO₃ (65% ig) eingesetzt wurden. Die Schnitthärte betrug 39 N (SA = 13 N).

Der Katalysator wies ein Splitt-Stampfgewicht von 0,858 g/ml auf. Die BET-Oberfläche betrug 124 m²/g. Durch Quecksilber-Porosimetrie-Messungen wurde ein Porenvolumen von 0,42 ml/g ermittelt und eine Porenfläche von 131 m²/g und ein mittlerer Porenradius von 10,1 nm errechnet.

Die Zusammensetzung des Katalysators ist Tabelle 1 zu entnehmen.

### Beispiel 4

Es wurde analog Beispiel 2 verfahren mit den Unterschieden, daß beim Verkneten 235,57 g des im ersten Schritt erhaltenen Sprühpulvers, 400 g Pural™ SCF (Böhmit, Glühverlust 25 %) und 12,71 g konz. HCOOH eingesetzt wurden. Die Schnitthärte betrug 27 N (SA = 12 N).

Der Katalysator wies ein Splitt-Stampfgewicht von 0,75 g/ml auf. Die BET-Oberfläche betrug 148 m²/g. Durch Quecksilber-Porosimetrie-Messungen wurde ein Porenvolumen von 0,49 ml/g ermittelt und eine Porenfläche von 169 m²/g und ein mittlerer Porenradius von 10,2 nm errechnet.

Die Zusammensetzung des Katalysators ist Tabelle 1 zu entnehmen.

### Beispiel 5

Es wurde analog Beispiel 2 verfahren mit den Unterschieden, daß beim Verkneten 471,14 g des im ersten Schritt erhaltenen Sprühpulvers, 72,16 g Puralox™ SCF (Al₂O₃, Glühverlust 3 %), 40 g Pural™ SB (Böhmit, Glühverlust 25 %) und 29,17 g konz. HNO₃ eingesetzt wurden. Die Schnitthärte betrug 28 N (SA = 9 N).

Der Katalysator wies ein Splitt-Stampfgewicht von 0,908 g/ml auf. Die BET-Oberfläche betrug 103 m²/g. Durch Quecksilber-Porosimetrie-Messungen wurde ein Porenvolumen von 0,39 ml/g ermittelt und eine Porenfläche von 99 m²/g und ein mittlerer Porenradius von 17,2 nm errechnet.

Die Zusammensetzung des Katalysators ist Tabelle 1 zu entnehmen.

### Beispiel 6

Es wurde analog Beispiel 2 verfahren mit den Unterschieden, daß beim Verkneten 500 g des im ersten Schritt erhaltenen Sprühpulvers und 50 g konz. HNO₃ ohne weitere Zusätze eingesetzt wurden. Die Schnitthärte betrug 27 N (SA = 9 N).

Der Katalysator wies ein Splitt-Stampfgewicht von 1,369 g/ml auf. Die BET-Oberfläche betrug 51 m²/g. Durch Quecksilber-Porosimetrie-Messungen wurde ein Porenvolumen von 0,17 ml/g ermittelt und eine Porenfläche von 88 m²/g und ein mittlerer Porenradius von 10,8 nm errechnet.

Die Zusammensetzung des Katalysators ist Tabelle 1 zu entnehmen.

### Beispiel 7

69,84 g eines zu 1,6 bis 2 mm gesplitteten ZrO₂ · xSiO₂-Trägers der Firma Norton (SiO₂-Gehalt 4,7%, Nr. 9816590) wurden mit einer Lösung, die 0,81196 g SnCl₂ · 2 H₂O und 0,5338 g H₂PtCl₆ · 6 H₂O in 412 ml Ethanol enthielt, getränkt. Die überstehende Lösung wurde i. Vak. entfernt und der Rückstand 15 h bei 100°C getrocknet und anschließend 3 h bei 560°C calciniert. Anschließend wurde mit einer Lösung von 0,5218 g CsNO₃ und 0,9204 g KNO₃ in 154 ml Wasser getränkt, der Katalysator 15 h bei 100°C getrocknet und anschließend 3 h bei 560°C calciniert.

Der Katalysator wies ein Splitt-Stampfgewicht von 1,139 g/ml auf. Die BET-Oberfläche betrug 81 m²/g. Durch Quecksilber-Porosimetrie-Messungen wurde ein Porenvolumen von 0,29 ml/g ermittelt und eine Porenfläche von 69 m²/g und ein mittlerer Porenradius von 12,1 nm errechnet.

Die Zusammensetzung des Katalysators ist Tabelle 1 zu entnehmen.

### Vergleichsbeispiel

Es wurde ein Katalysator nach der Vorschrift der WO-A-94/29021, Beispiel 1 zum Vergleich präpariert (Pt/Sn/Cs/Mg(Al)O).

### Katalysatortest

20 ml des Katalysators wurden in einen Rohrreaktor mit einem Innendurchmesser von 22 mm eingebaut. Der Katalysator wurde bei einer Temperatur von 580°C 30 min mit Wasserstoff versetzt, danach einem Gemisch aus 80% Stickstoff und 20% Luft (Magerluft) ausgesetzt, anschließend 15 min mit reinem Stickstoff versetzt, 30 min mit Wasserstoff reduziert und mit 20 Nl/h Propan (99,5 %ig) und H₂O im Molverhältnis Propan/Wasserdampf von 1 : 1 beaufschlagt. Der Druck betrug 1,5 bar, die GHSV betrug 1000 h⁻¹. Die Reaktionsprodukte wurden gaschromatographisch erfaßt.

Die Ergebnisse mit den Katalysatoren der Beispiele 1 bis 7 und des Vergleichsbeispiels sind in Tabelle 1 aufgeführt.

**Tabelle 1**

| Performance der Katalysatoren der Beispiele 1 bis 7 und des Vergleichsbeispiels in der Propan-Dehydrierung* | | | | | | | | | | | |
|---|---|---|---|---|---|---|---|---|---|---|---|
| | | | | | | | | Umsatz [%] nach | | Selektivität [%] nach | |
| Beispiel Nr.: | Pt [%] | Sn [%] | K [%] | Cs [%] | ZrO₂ [%] | SiO₂ [%] | Al₂O₃ [%] | 1 h | 17 h | 1 h | 17 h |
| 1 | 0,3 | 0,3 | 0,5 | 0,5 | 76,0 | 2,8 | 19,6 | 36 | 33 | 89 | 95 |
| 2 | 0,3 | 0,3 | 0,5 | 0,5 | 73,5 | 5,5 | 19,6 | 38 | 33 | 94 | 87 |
| 3 | 0,3 | 0,3 | 0,5 | 0,5 | 54,9 | 4,1 | 39,4 | 42 | 35 | 87 | 86 |
| 4 | 0,3 | 0,3 | 0,5 | 0,5 | 36,6 | 2,7 | 59,1 | 35 | 29 | 78 | 91 |
| 5 | 0,3 | 0,3 | 0,5 | 0,5 | 73,2 | 5,5 | 19,7 | 39 | 31 | 90 | 91 |
| 6 | 0,3 | 0,3 | 0,5 | 0,5 | 94,9 | 3,5 | --- | 33 | 31 | 96 | 97 |
| 7 | 0,3 | 0,3 | 0,5 | 0,5 | 93,8 | 4,6 | --- | 37 | 34 | 85 | 97 |
| Vergleich | 0,3 | 0,3 | | 0,5 | --- | --- | --- | 33 | 29 | 92 | 95 |

| | | | | | | | | | | | |
|---|---|---|---|---|---|---|---|---|---|---|---|
| *) Versuchsbedingungen: 20 ml Katalysator, Splittgröße 1,6 bis 2 mm; 580°C; Propan/H₂O 1 : 1 (mol/mol); 20 Nl/h Propan; GHSV = 1000 h⁻¹; 1,5 bar. | | | | | | | | | | | |
| **) Vergleichskatalysator Pt/Sn/Cs/Mg(Al)O aus WO-A-94/29021 Beispiel 1. | | | | | | | | | | | |

### Beispiel 8

Der in Beispiel 1 hergestellte Katalysator wurde für die Isobutan-Dehydrierung getestet. Es wurde dabei vorgegangen wie in Beispiel 1 beschrieben, mit dem Unterschied, daß statt Propan Isobutan eingesetzt wurde.

**Tabelle 2**

| Performance des Katalysators gemäß Beispiel 1 in der Isobutan-Dehydrierung* | | | | | | | | | | | |
|---|---|---|---|---|---|---|---|---|---|---|---|
| | | | | | | | | Umsatz [%] nach | | Selektivität [%] nach | |
| Beispiel Nr.: | Pt [%] | Sn [%] | K [%] | Cs [%] | ZrO₂ [%] | SiO₂ [%] | Al₂O₃ [%] | 1 h | 17 h | 1 h | 17 h |
| 8 | 0,3 | 0,3 | 0,5 | 0,5 | 73,7 | 5,6 | 19,1 | 54 | 54 | 89 | 92 |

| | | | | | | | | | | | |
|---|---|---|---|---|---|---|---|---|---|---|---|
| *) Versuchsbedingungen: 20 ml Katalysator, Splittgröße 1,6 bis 2 mm; 580°C; Isobutan/H₂O 1 : 1 (mol/mol); 20 Nl/h Isobutan; GHSV = 1000 h⁻¹; 1,5 bar. | | | | | | | | | | | |

## Patentansprüche

1. Katalysatoren enthaltend
a) 10 bis 99,9 Gew.-% Zirkondioxid und/oder Titandioxid und
b) 0,1 bis 30 Gew.-% Siliciumdioxid und
c) 0 bis 60 Gew.-% Aluminiumoxid und
d) 0,1 bis 10 Gew.-% mindestens eines Elementes der ersten oder zweiten Hauptgruppe, eines Elementes der dritten Nebengruppe, eines Elementes der achten Nebengruppe des Periodensystems der Elemente und/oder Zinn,
mit der Maßgabe, daß die Summe der Gewichtsprozente 100 ergibt.

2. Katalysatoren bestehend im Wesentlichen aus
a) 10 bis 99,9 Gew.-% Zirkondioxid und/oder Titandioxid und
b) 0,1 bis 30 Gew.-% Siliciumdioxid und
c) 0 bis 60 Gew.-% Aluminiumoxid und
d) 0,1 bis 10 Gew.-% mindestens eines Elementes der ersten oder zweiten Hauptgruppe, eines Elementes der dritten Nebengruppe, eines Elementes der achten Nebengruppe des Periodensystems der Elemente und/oder Zinn,
mit der Maßgabe, daß die Summe der Gewichtsprozente 100 ergibt.

3. Katalysatoren nach einem der Ansprüche 1 oder 2, dadurch gekennzeichnet, daß diese 0,1 bis 5 Gew.-% Kalium und/oder Cäsium enthalten.

4. Katalysatoren nach einem der Ansprüche 1 bis 3, dadurch gekennzeichnet, daß diese 0,05 bis 1 Gew.-% Platin und 0,05 bis 2 Gew.-% Zinn enthalten.

5. Katalysatoren nach einem der Ansprüche 1 bis 4, gekennzeichnet dadurch, daß diese eine BET-Oberfläche zwischen 40 und 250 m²/g, ein Porenvolumen zwischen 0,2 und 0,5 ml/g und einen mittleren Porenradius zwischen 8 und 20 nm aufweisen.

6. Verwendung der Katalysatoren nach einem der Ansprüche 1 bis 5 zur Dehydrierung von C₂- bis C₁₆-Kohlenwasserstoffen.

7. Verfahren zur Dehydrierung von C₂- bis C₁₆-Kohlenwasserstoffen gegebenenfalls in Gegenwart von Wasserdampf und eines Katalysators nach einem der Ansprüche 1 bis 5.

8. Verfahren zur Herstellung von Katalysatoren nach den Ansprüchen 1 bis 5, dadurch gekennzeichnet, daß man geeignete Verbindungen der Elemente Zirkon und/oder Titan und Aluminium und/oder Silicium
a) trocken mischt oder
b) gemeinsame versprüht
und anschließend calciniert und gegebenenfalls unter Zusatz von Säure zu einem Formkörper verarbeitet.

9. Verfahren zur Herstellung der Katalysatoren nach den Ansprüchen 1 bis 5, dadurch gekennzeichnet, daß man
a) geeignete Verbindungen der Elemente Zirkon und/oder Titan und Silicium versprüht oder trocken mischt und anschließend calciniert,
b) mit geeigneten Verbindungen des Aluminiums unter Zusatz einer Säure vermischt,
c) unter Zusatz von Säure in einen Formkörper überführt,
d) mit einer Platin- und Zinn-haltigen Lösung tränkt, trocknet und bei Temperaturen von 400 bis 650°C calciniert,
e) mit einer Kalium- und Cäsium-haltigen wäßrigen Lösung tränkt, trocknet und bei Temperaturen von 400 bis 650°C calciniert.

10. Verfahren nach Anspruch 8 oder 9, dadurch gekennzeichnet, daß man als Säure Salpetersäure oder Ameisensäure zusetzt.
